# EUROPÄISCHE PATENTSCHRIFT

(11) **EP 1 518 491 B1**
(45) Veröffentlichungstag und Bekanntmachung des Hinweises auf die Patenterteilung: **10.04.2013**
(21) Anmeldenummer: 04022733.2
(22) Anmeldetag: 23.09.2004
(51) Int. Cl.: A61B 1/00

(54) **Adapter zum Verbinden einer Kamera mit einem medizinischen Gerät**
Adapter for connecting a camera with a medical endoscope
Adaptateur pour relier une caméra à un endoscope médical

(30) Priorität: 23.09.2003 DE 20314780 U
(43) Veröffentlichungstag der Anmeldung: 30.03.2005
(73) Patentinhaber: Udo Heisig GmbH, 85640 München (DE)
(72) Erfinder: Heisig, Udo, 85640 Putzbrunn (DE)
(74) Vertreter: Feldkamp, Rainer

(56) Entgegenhaltungen:
- EP-A- 0 809 976
- EP-A2- 0 200 339
- DE-A- 3 341 876
- DE-A- 3 541 855
- DE-U- 9 413 384
- DE-U- 29 616 666
- US-A- 4 522 196
- US-A- 4 778 252
- US-A- 5 707 340
- US-B1- 6 346 073

## Beschreibung

Die Erfindung bezieht sich auf einen Adapter zum Verbinden einer Kamera mit einem medizinischen Gerät, beispielsweise einem Endoskop oder einer Optik, der im Oberbegriff des Anspruchs 1 genannten Art.

Derartige Kameras weisen Anschlussleitungen auf , die ebenso wie die Kamera nur schwierig oder überhaupt nicht sterilisiert werden können und daher vor Gebrauch mit einer sterilen Folienschlauchabdeckung überzogen werden müssen. Das Objektiv der Kamera wird daher über einen Adapter lösbar mit einem flanschförmigen Ende des medizinischen Gerätes verbunden, wobei der Adapter gleichzeitig zur Befestigung eines Endes der Folienschlauchabdeckung dient.

Ein derartiger Adapter ist aus dem US-Patent 5 882 295 bekannt. Bei diesem bekannten Adapter sind nicht näher erläuterte Verriegelungselemente vorgesehen, die das flanschförmige Ende des medizinischen Gerätes in einer Steckbuchse des Adapters festlegen können.

Zur Festlegung des flanschförmigen Endes des medizinischen Gerätes sind die verschiedensten Ausführungen bekannt, wie Klemmschieber, Überwurfhülsen und dergleichen, die alle zu einem komplizierten und aufwendigen Aufbau des Adapters führen.

Aus dem Dokument DE 296 16 666 U1 ist ein Adapter bekannt gemäß dem Oberbegriff des Anspruchs 1, bei dem sich von einer Grundfläche eines Grundkörpers elastische Zungen erstrecken, zwischen denen ein flanschförmiges Ende eines medizinischen Gerätes, beispielsweise eines Endoskops angeordnet werden kann, wobei diese elastischen Zungen von einer Ringhülse umgeben sind, die sich rampenförmig radial nach innen erstreckende Vorsprünge aufweist, so dass bei einer Verdrehung der Ringhülse gegenüber den elastischen Zungen diese gegen das flanschförmige Ende vorgespannt werden. Die Ringhülse ist hierbei lediglich durch die elastischen Zungen an dem Grundkörper gehaltert, so dass bei Auftreten von Querkräften in dem Adapter eine Verschiebung der optischen Achsen der durch den Adapter zu verbindenden Teile auftritt.

Der Erfindung liegt die Aufgabe zugrunde, einen Adapter der eingangs genannten Art zu schaffen, der bei einfachem Aufbau eine sichere und sterile Verbindung der Kamera mit dem medizinischen Gerät ermöglicht.

Vorteilhafte Ausgestaltungen und Weiterbildungen der Erfindung ergeben sich aus den Unteransprüchen.

Die Erfindung wird nachfolgend anhand der Zeichnungen noch näher erläutert.

In der Zeichnung zeigen:
Figur 1 eine perspektivische Ansicht des Adapters von der Seite der Steckbuchse für das medizinische Gerät.
Figur 2 eine perspektivische Ansicht des Adapters von der Seite des Kupplungsringes für die Kamera.
Figur 3 eine Seitenansicht des Adapters.
Figur 4 eine Draufsicht auf den Adapter nach Figur 1.
Figur 5 eine Schnittansicht des Adapters entlang der Schnittinie A-A nach Figur 4
Figur 6 die in Figur 5 mit B bezeichnete Einzelheit in vergrösserter Darstellung.

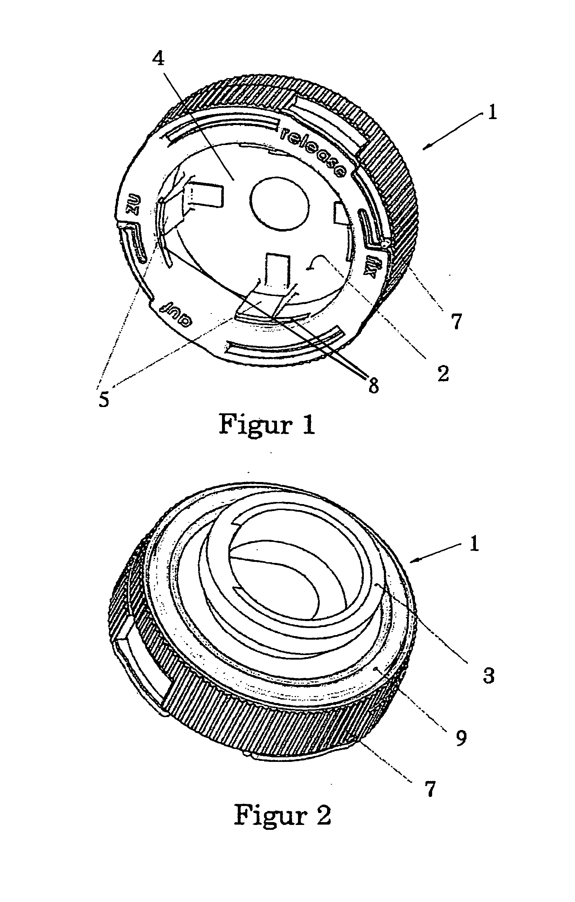

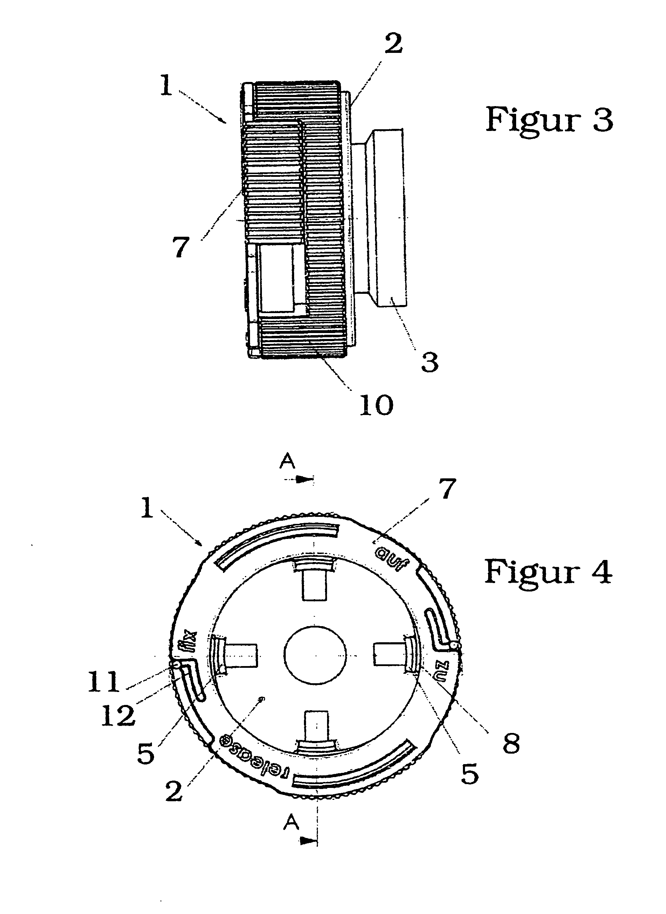

In den Figuren 1 bis 3 sind Aussenansichten des Adapters gemäß einer Ausführungsform der Erfindung gezeigt.

Der Adapter 1 dient zum Verbinden einer (nicht dargestellten) Kamera mit einem (ebenfalls nicht dargestellten) medizinischen Gerät, beispielsweise einem Endoskop oder einer Optik, wie dies beispielsweise in dem US-Patent 5 882 295 gezeigt ist.

Der Adapter weist eine im Wesentlichen kreisförmige Grundplatte 2 auf, die auf einer ersten Hauptfläche einen Kupplungsring 3 zum Anschluss der Kamera und auf der der ersten Hauptfläche gegenüberliegenden zweiten Hauptfläche eine Steckbuchse 4 zum Anschluss eines flanschförmigen Endes des medizinischen Gerätes aufweist, wobei Verriegelungselemente am freien Ende der Steckbuchse ein Festlegen des flanschförmigen Endes in der Steckbuchse ermöglichen.

Diese Verriegelungselemente sind durch eine Anzahl von sich senkrecht zur Ebene der zweiten Hauptfläche erstreckenden elastischen Zungen 5 mit radial nach innen gerichteten Rastkanten 6 gebildet, die am Aussenumfang von einer Ringhülse 7 umgeben sind.

Diese Ringhülse 7 ist gegenüber den Zungen 5 und der Grundplatte 2 um eine sich senkrecht zur Grundplatte 2 erstreckende Achse drehbar, wobei die Ringhülse 7 im Bereich der freien Enden der Zungen 5 sich in Umfangsrichtung erstreckende und rampenförmig radial nach innen vorspringende Vorsprünge 8 aufweist, die bei einer Verdrehung der Ringhülse 7 gegenüber der Grundplatte in einer ersten Richtung mit dem Aussenumfang der Zungen 5 in Eingriff kommen und diese zunehmend radial nach innen drücken, so dass ein in dem von den Zungen 5 umgrenzten Innenbereich anzuordnendes flanschförmiges Ende des medizinischen Gerätes zwischen der Grundplatte 2 und den Rastkanten 6 der Zungen eingeklemmt wird.

Auf diese Weise kann das flanschförmige Ende des medizinischen Gerätes sicher in der Steckbuchse festgelegt werden.

Die Grundplatte 2 weist einen sich in Axialrichtung von der zweiten Hauptfläche fort erstreckenden Umfangsflansch 10 zur Führung der Drehbewegung der Ringhülse 7 aufweist.

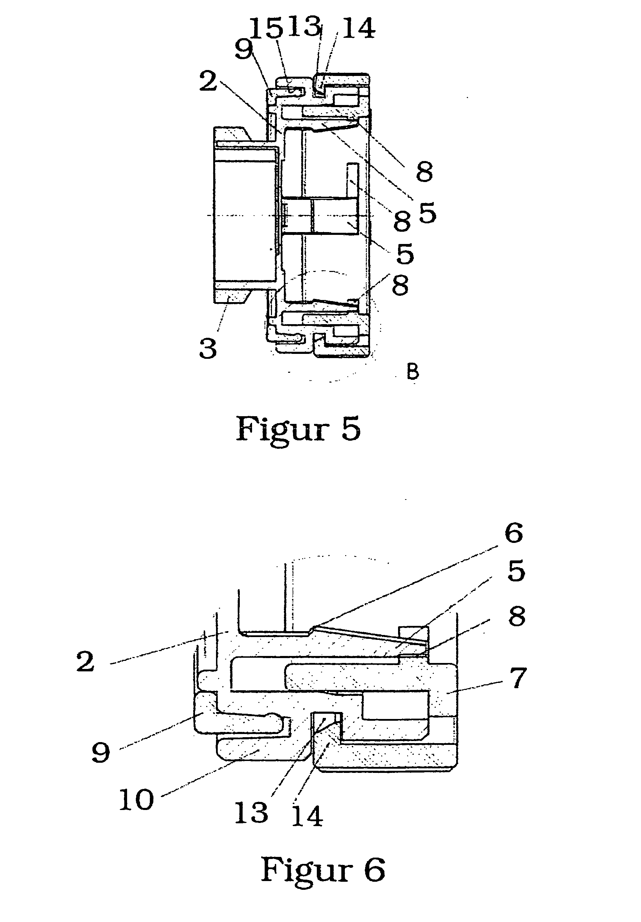

Die Ringhülse weist in der insbesondere aus Figur 6 ersichtlichen Weise einen Aussenringteil und einen Innenringteil auf, die am freien Ende miteinander verbunden sind und zwischen sich eine Nut bilden, in die der Umfangsflansch eingreifen kann. Der Innenringteil trägt hierbei die Rampen 8.

Der Umfangsflansch 10 der Grundplatte 2 und die Ringhülse 7 weisen ineinandergreifende Befestigungselemente 13, 14 auf, die die Ringhülse 7 in Axialrichtung gegenüber der Grundplatte 2 festlegen, jedoch eine freie Drehung der Ringhülse 7 ermöglichen.

Diese Befestigungselemente können durch eine Ringnut 13 am Aussenumfang des Umfangsflansches 10 und eine Umbördelung 14 an dem der zweiten Hauptfläche zugewandten Umfangsrand der Ringhülse gebildet sein, die in die Ringnut 13 einrasten kann.

Die Zungen 5 sind vorzugsweise einstückig mit der Grundplatte (2) ausgebildet.

Die Grundplatte 2 ist in der ersten Hauptfläche mit einer Ringnut 15 versehen, in die ein Klemmring 9 einrastbar ist, wobei zwischen dem Klemmring 9 und der Grundplatte 2 ein Ende einer Folienschlauchabdeckung einklemmbar ist, die die Kamera und deren Anschlussleitungen abdeckt.

An dem Umfangsflansch 10 und der Ringhülse 7 sind vorzugsweise elastische Rasteinrichtungen 11 bzw. 12 zur Festlegung einer ersten Drehstellung der Ringhülse 7, in der die rampenförmigen Vorsprünge 8 die Zungen 5 freigeben und das Einsetzen des flanschförmigen Endes des medizinischen Gerätes ermöglichen, und einer zweiten Drehstellung angeordnet, in der die Vorsprünge 8 die Zungen 5 über das flanschförmige Ende verformen und dieses mit ihren Rastkanten 6 festhalten.

Auf diese Weise ergibt sich ein einfacher Aufbau des Adapters aus drei im Spritzgussverfahren herstellbaren Teilen, nämlich der Grundplatte, die den Kupplungsring, den Umfangsflansch und die Zungen trägt, sowie der Ringhülse und dem Klemmring. Alle diese Teile werden durch einfaches Einrasten aneinder befestigt und ermöglichen weiterhin eine schnelle und sichere Befestigung eines Endes der Folienschlauchabdeckung, die die Kamera und deren Anschlussleitungen abdeckt.

## Patentansprüche

1. Adapter (1) zum Verbinden einer Kamera mit einem medizinischen Gerät, beispielsweise einem Endoskop oder einer anderen Optik, wobei der Adapter eine Grundplatte (2) aufweist, die auf einer ersten Hauptfläche einen Kupplungsring (3) zum Anschluss einer Kamera und auf der der ersten Hauptfläche gegenüberliegenden zweiten Hauptfläche eine Steckbuchse (4) zum Anschluss eines flanschförmigen Endes des medizinischen Gerätes aufweist, wobei Verriegelungselemente am freien Ende der Steckbuchse ein Festlegen des flanschförmigen Endes der Steckbuchse ermöglichen und eine Anzahl von sich senkrecht zur Ebene der zweiten Hauptfläche der Grundplatte (2) erstreckenden elastischen Zungen (5) mit radial nach innen gerichteten Rastkanten (6) umfassen, wobei die Zungen (5) am Aussenumfang von einer Ringhülse (7) umgeben sind, die gegenüber den Zungen (5) und der Grundplatte (2) um eine sich senkrecht zur Grundplatte erstreckende Achse drehbar ist und im Bereich der freien Enden der Zungen (5) sich in Umfangsrichtung erstreckende und rampenförmig radial nach innen vorspringende Vorsprünge (8) aufweist, die bei einer Verdrehung der Ringhülse (7) gegenüber der Grundplatte in einer ersten Richtung mit dem Aussenumfang der Zungen (5) in Eingriff kommen und diese zunehmend radial nach innen drücken, so dass der in dem von den Zungen (5) umgrenzte Innenbereich anzuordnende Ringflansch des medizinischen Gerätes zwischen der Grundplatte (2) und den Rastkanten (6) der Zungen eingeklemmt wird,
**dadurch gekennzeichnet, dass** die Grundplatte (2) einen sich in Axialrichtung von der zweiten Hauptfläche fort erstreckenden Umfangsflansch (10) zur Führung der Drehbewegung der Ringhülse (7) aufweist, und dass der Umfangsflansch (10) der Grundplatte (2) und die Ringhülse (7) ineinandergreifende Befestigungselemente (13, 14) aufweisen, die die Ringhülse (7) in Axialrichtung gegenüber der Grundplatte (2) festlegen, jedoch eine Drehung der Ringhülse (7) ermöglichen.

2. Adapter nach Anspruch 1, **dadurch gekennzeichnet, dass** die Befestigungselemente (13, 14) durch eine Ringnut (13) am Aussenumfang des Umfangsflansches (10) und eine Umbördelung (14) an dem der zweiten Hauptfläche zugewandten Umfangsrand der Ringhülse (7) gebildet sind, die in die Ringnut (13) einrastbar ist.

3. Adapter nach Anspruch 1 oder 2, **dadurch gekennzeichnet, dass** der Umfangsflansch (10) und die Ringhülse (7) elastische Rasteinrichtungen (11, 12) zur Festlegung einer ersten Drehstellung der Ringhülse (7), in der die rampenförmigen Vorsprünge (8) die Zungen (5) freigeben und das Einsetzen des flanschförmigen Endes des medizinischen Gerätes ermöglichen, und einer zweiten Drehstellung aufweisen, in der die Vorsprünge (8) die Zungen (5) über das flanschförmige Ende verformen und dieses mit ihren Rastkanten festhalten.

4. Adapter nach einem der vorhergehenden Ansprüche, **dadurch gekennzeichnet, dass** die Grundplatte (2) in der ersten Hauptfläche eine Ringnut (15) aufweist, in die ein Klemmring (9) einrastbar ist, und dass zwischen dem Klemmring (9) und der Grundplatte (2) ein Ende einer Folienschlauchabdeckung einklemmbar ist, die die Kamera und deren Anschlussleitungen abdeckt.

## Claims

1. An adaptor (1) for connecting a camera to a medical device, for example an endoscope or another optical device, wherein the adaptor has a base plate (2) which on a first main surface has a coupling ring (3) for the connection of a camera and on the second main surface opposite to the first main surface has a connection socket (4) for the connection of a flange-shaped end of the medical device, wherein locking elements at the free end of the connection socket permit fixing of the flange-shaped end of the connection socket and include a number of elastic tongues (5) which extend perpendicularly relative to the plane of the second main surface of the base plate (2) and which have radially inwardly directed latching edges (6), wherein the tongues (5) are surrounded at the outer periphery by an annular sleeve (7) which is rotatable relative to the tongues (5) and the base plate (2) about an axis extending perpendicularly to the base plate and in the region of the free ends of the tongues (5) has projections (8) which extend in the peripheral direction and which project radially inwardly in a ramp shape and which upon a rotary movement of the annular sleeve (7) with respect to the base plate in a first direction come into engagement with the outer periphery of the tongues (5) and urge them increasingly radially inwardly so that the annular flange of the medical device, that is to be arranged in the inside region surrounded by the tongues (5), is clamped between the base plate (2) and the latching edges (6) of the tongues,
**characterised in that** the base plate (2) has a peripheral flange (10) extending in the axial direction away from the second main surface for guiding the rotary movement of the annular sleeve (7) and the peripheral flange (10) of the base plate (2) and the annular sleeve (7) have securing elements (13, 14) which engage into each other and which fix the annular sleeve (7) in the axial direction relative to the base plate (2) but permit a rotary movement of the annular sleeve (7).

2. An adaptor according to claim 1 **characterised in that** the securing elements (13, 14) are formed by an annular groove (13) at the outside periphery of the annular flange (10) and a flanged-over portion (14) at the peripheral edge of the annular sleeve (7) facing towards the second main surface, that can be latched into the annular groove (13).

3. An adaptor according to claim 1 or claim 2 **characterised in that** the peripheral flange (10) and the annular sleeve (7) have elastic latching devices (11, 12) for fixing a first rotary position of the annular sleeve (7), in which the ramp-shaped projections (8) release the tongues (5) and permit insertion of the flange-shaped end of the medical device, and a second rotary position in which the projections (8) deform the tongues (5) over the flange-shaped end and secure same with their latching edges.

4. An adaptor according to one of the preceding claims **characterised in that** in the first main surface the base plate (2) has an annular groove (15) into which a clamping ring (9) is latchable, and an end of a film tube cover which covers the camera and the connecting lines thereof can be clamped between the clamping ring (9) and the base plate (2).

## Revendications

1. Adaptateur (1) permettant de relier une caméra à un appareil médical, par exemple à un endoscope ou à un autre appareil optique, dans lequel l'adaptateur présente une plaque de base (2) qui présente sur une première surface principale un anneau d'accouplement (3) destiné au raccordement d'une caméra, et sur la seconde surface principale opposée à la première surface principale une fiche femelle (4) destinée au raccordement d'une extrémité en forme de bride de l'appareil médical, où des éléments de verrouillage au niveau de l'extrémité libre de la fiche femelle permettent une fixation de l'extrémité en forme de bride de la fiche femelle, et où un nombre de languettes élastiques (5) qui s'étendent perpendiculairement au plan de la seconde surface principale de la plaque de base (2) comprennent des arêtes d'encliquetage (6) orientées radialement vers l'intérieur, où les languettes (5) sont entourées au niveau de la périphérie extérieure par une douille annulaire (7) qui peut pivoter par rapport aux languettes (5) et à la plaque de base (2) autour d'un axe qui s'étend perpendiculairement à la plaque de base et qui présente dans la zone de l'extrémité libre des languettes (5) des saillies (8) qui s'étendent dans la direction périphérique et dépassent radialement en rampe vers l'intérieur, lesquelles saillies lors d'une rotation de la douille annulaire (7) par rapport à la plaque de base viennent en prise dans une première direction avec la périphérie extérieure des languettes (5) et les compriment davantage radialement vers l'intérieur, de sorte que la bride annulaire de l'appareil médical agencée dans la zone interne délimitée par les languettes (5) est serrée ou pincée entre la plaque de base (2) et les arêtes d'encliquetage (6),
**caractérisé en ce que** la plaque de base (2) présente une bride périphérique (10) qui se prolonge dans la direction axiale depuis la seconde surface principale pour guider le déplacement en rotation de la douille annulaire (7), et **en ce que** la bride périphérique (10) de la plaque de base (2) et la douille annulaire (7) présentent des éléments de fixation s'insérant les uns dans les autres (13, 14), lesquels fixent la douille annulaire (7) dans la direction axiale par rapport à la plaque de base (2) mais autorisent cependant une rotation de la douille annulaire (7).

2. Adaptateur selon la revendication 1, **caractérisé en ce que** les éléments de fixation (13, 14) sont formés par une rainure annulaire (13) au niveau de la périphérie extérieure de la bride périphérique (10) et un rabat ou jupe (14) au niveau du bord périphérique de la douille annulaire (7) orienté vers la seconde surface principale, qui peut être encliqueté dans la rainure annulaire (13).

3. Adaptateur selon la revendication 1 ou 2, **caractérisé en ce que** la bride périphérique (10) et la douille annulaire (7) présentent des dispositifs d'encliquetage élastiques (11, 12) pour fixer une première position de rotation de la douille annulaire (7) dans laquelle les saillies en forme de rampe (8) libèrent les languettes (5) et permettent le placement de l'extrémité en forme de bride de l'appareil médical, et une seconde position de rotation dans laquelle les saillies (8) déforment les languettes (5) au-delà de l'extrémité en forme de bride et la fixe par leurs arêtes d'encliquetage.

4. Adaptateur selon l'une des revendications précédentes, **caractérisé en ce que** la plaque de base (2) présente dans la première surface principale une rainure annulaire (15) dans laquelle peut s'encliqueter un anneau de serrage (9), et **en ce qu'**entre l'anneau de serrage (9) et la plaque de base (2) une extrémité d'un cache en tube soufflé, qui protège la caméra et ses conduites de raccordement, peut être serrée ou pincée.
